# EUROPEAN PATENT SPECIFICATION

(11) **EP 2 799 546 B1**
(45) Date of publication and mention of the grant of the patent: **12.07.2017**
(21) Application number: 12862230.5
(22) Date of filing: 09.05.2012
(51) Int. Cl.: C12N 15/11, G01N 33/53, G01N 33/68, C12Q 1/68

(54) **RADIATION EXPOSURE DIAGNOSTIC MARKER IGFBP-5 AND METHOD FOR DIAGNOSING RADIATION EXPOSURE BY MEASURING THE EXPRESSION LEVEL OF THE MARKER**
STRAHLENEXPOSITIONSDIAGNOSEMARKER IGFBP-5 UND VERFAHREN ZUR DIAGNOSE EINER STRAHLENEXPOSITION DURCH MESSUNG DES AUSDRUCKSNIVEAUS DES MARKERS
MARQUEUR DIAGNOSTIQUE D'EXPOSITION À UN RAYONNEMENT IGFBP-5 ET MÉTHODE PERMETTANT DE DIAGNOSTIQUER L'EXPOSITION À UN RAYONNEMENT PAR MESURE DU NIVEAU D'EXPRESSION DU MARQUEUR

(30) Priority: 30.12.2011 KR 20110147425
(43) Date of publication of application: 05.11.2014
(73) Proprietor: Korea Institute of Radiological & Medical Sciences, Nowon-gu, Seoul 139-240 (KR)
(72) Inventor: KIM, Kwang Seok, Goyang-si Gyeonggi-do 410-721 (KR); BAE, Sang Woo, Seoul 139-230 (KR); CHOI, Kyu Jin, Seoul 137-070 (KR); KIM, Eun Sook, Seoul 139-786 (KR)
(74) Representative: Potter Clarkson LLP
(86) International application number: PCT/KR2012/003604
(87) International publication number: WO 2013/100273

(56) References cited:
- WO-A1-03/006029
- KR-B1- 100 458 364
- A. J. BUTT ET AL: "Insulin-like Growth Factor-binding Protein-5 Inhibits the Growth of Human Breast Cancer Cells in Vitro and in Vivo", JOURNAL OF BIOLOGICAL CHEMISTRY, vol. 278, no. 32, 30 May 2003 (2003-05-30) , pages 29676-29685, XP55155962, ISSN: 0021-9258, DOI: 10.1074/jbc.M301965200
- TANNO B ET AL: "Silencing of endogenous IGFBP-5 by micro RNA interference affects proliferation, apoptosis and differentiation of neuroblastoma cells", CELL DEATH AND DIFFERENTIATION, NATURE PUBLISHING GROUP, GB, vol. 12, no. 3, 1 March 2005 (2005-03-01), pages 213-223, XP002631062, ISSN: 1350-9047, DOI: 10.1038/SJ.CDD.4401546 [retrieved on 2004-12-24]
- TUSHER V G ET AL: "Significance analysis of microarrays applied to the ionizing radition response", PROCEEDINGS OF THE NATIONAL ACADEMY OF SCIENCES, NATIONAL ACADEMY OF SCIENCES, US, vol. 98, no. 9, 24 April 2001 (2001-04-24) , pages 5116-5121, XP002967440, ISSN: 0027-8424, DOI: 10.1073/PNAS.091062498
- KIS E ET AL: "Microarray analysis of radiation response genes in primary human fibroblasts", INTERNATIONAL JOURNAL OF RADIATION: ONCOLOGY BIOLOGY PHYSICS, PERGAMON PRESS, USA, vol. 66, no. 5, 1 December 2006 (2006-12-01), pages 1506-1514, XP024899639, ISSN: 0360-3016, DOI: 10.1016/J.IJROBP.2006.08.004 [retrieved on 2006-12-01]
- BUTT ET AL.: 'Insulin-like growth factor-binding protein-5 inhibits the growth of human breast cancer cells in vitro and in vivo' THE JOURNAL OF BIOLOGICAL CHEMISTRY vol. 278, no. 32, 08 August 2003, pages 29676 - 29685, XP055155962
- KIS ET AL.: 'Microarray analysis of radiation response genes in primary human fibroblasts' INTERNATIONAL JOURNAL OF RADIATION ONCOLOGY BIOLOGY PHYSICS vol. 66, no. 5, 2006, pages 1506 - 1514, XP024899639
- OTOMO ET AL.: 'Microarray analysis of temporal gene responses to ionizing radiation in two glioblastoma cell lines: up-regulation of DNA repair genes' JOURNAL OF RADIATION RESEARCH vol. 45, 2004, pages 53 - 60, XP055155963
- TUSHER ET AL.: 'Significance analysis of microarryas applied to the ionizing radiation response' PNAS vol. 98, no. 9, 24 April 2001, pages 5116 - 5121, XP002967440

<110> KOREA INSTITUTE OF RADIOLOGICAL & MEDICAL SCIENCES
<120> IGFBP-5, a marker for diagnosing radiation exposure, a composition for diagnosing radiation exposure to measure the level of expression of the marker, a kit for diagnosing radiation exposure comprising the composition, and a method for diagnosing radiation exposure using the marker
<130> pn095170
<160> 2
<170> KopatentIn 1.71
<210> 1
   <211> 6333
   <212> DNA
   <213> Homo sapiens
<400> 1
   gtagtctctt tggaaacttc tgcaggggaa aagagctagg aaagagctgc aaagcagtgt      60
   gggctttttc ccttttttgc tccttttcat tacccctcct ccgttttcac ccttctccgg     120
   acttcgcgta gaacctgcga atttcgaaga ggaggtggca aagtgggaga aaagaggtgt     180
   tagggtttgg ggtttttttg tttttgtttt tgttttttaa tttcttgatt tcaacatttt     240
   ctcccaccct ctcggctgca gccaacgcct cttacctgtt ctgcggcgcc gcgcaccgct     300
   ggcagctgag ggttagaaag cggggtgtat tttagatttt aagcaaaaat tttaaagata     360
   aatccatttt tctctcccac ccccaacgcc atctccactg catccgatct cattatttcg     420
   gtggttgctt gggggtgaac aattttgtgg ctttttttcc cctataattc tgacccgctc     480
   aggcttgagg gtttctccgg cctccgctca ctgcgtgcac ctggcgctgc cctgcttccc     540
   ccaacctgtt gcaaggcttt aattcttgca actgggacct gctcgcaggc accccagccc     600
   tccacctctc tctacatttt tgcaagtgtc tgggggaggg cacctgctct acctgccaga     660
   aattttaaaa caaaaacaaa aacaaaaaaa tctccggggg ccctcttggc ccctttatcc     720
   ctgcactctc gctctcctgc cccaccccga ggtaaagggg gcgactaaga gaagatggtg     780
   ttgctcaccg cggtcctcct gctgctggcc gcctatgcgg ggccggccca gagcctgggc     840
   tccttcgtgc actgcgagcc ctgcgacgag aaagccctct ccatgtgccc ccccagcccc     900
   ctgggctgcg agctggtcaa ggagccgggc tgcggctgct gcatgacctg cgccctggcc     960
   gaggggcagt cgtgcggcgt ctacaccgag cgctgcgccc aggggctgcg ctgcctcccc    1020
   cggcaggacg aggagaagcc gctgcacgcc ctgctgcacg gccgcggggt ttgcctcaac    1080
   gaaaagagct accgcgagca agtcaagatc gagagagact cccgtgagca cgaggagccc    1140
   accacctctg agatggccga ggagacctac tcccccaaga tcttccggcc caaacacacc    1200
   cgcatctccg agctgaaggc tgaagcagtg aagaaggacc gcagaaagaa gctgacccag    1260
   tccaagtttg tcgggggagc cgagaacact gcccaccccc ggatcatctc tgcacctgag    1320
   atgagacagg agtctgagca gggcccctgc cgcagacaca tggaggcttc cctgcaggag 1380
   ctcaaagcca gcccacgcat ggtgccccgt gctgtgtacc tgcccaattg tgaccgcaaa 1440
   ggattctaca agagaaagca gtgcaaacct tcccgtggcc gcaagcgtgg catctgctgg 1500
   tgcgtggaca agtacgggat gaagctgcca ggcatggagt acgttgacgg ggactttcag 1560
   tgccacacct tcgacagcag caacgttgag tgatgcgtcc ccccccaacc tttccctcac 1620
   cccctcccac ccccagcccc gactccagcc agcgcctccc tccaccccag gacgccactc 1680
   atttcatctc atttaaggga aaaatatata tctatctatt tgaggaaact gaggacctcg 1740
   gaatctctag caagggctca acttcgaaaa tggcaacaac agagatgcaa aaagctaaaa 1800
   agacaccccc cccctttaaa tggttttctt tttgaggcaa gttggatgaa cagagaaggg 1860
   aagagaggaa gaacgagagg aagagaaggg aaggaagtgt ttgtgtagaa gagagagaaa 1920
   gacgaataga gttaggaaaa ggaagacaag caggtgggca ggaaggacat gcaccgagac 1980
   caggcagggg cccaactttc acgtccagcc ctggcctggg gtcgggagag gtgggcgcta 2040
   gaagatgcag cccaggatgt ggcaatcaat gacactattg gggtttccca ggatggattg 2100
   gtcaggggga gaaaggaaaa ggcaaaacac tccaggacct ctcccggatc tgtctcctcc 2160
   tctagccagc agtatggaca gctggacccc tgaacttcct ctcctcttac ctgggcagag 2220
   tgttgtctct ccccaaattt ataaaaacta aaatgcattc cattcctctg aaagcaaaac 2280
   aaattcataa ttgagtgata ttaaatagag aggttttcgg aagcagatct gtgaatatga 2340
   aatacatgtg catatttcat tccccaggca gacatttttt agaaatcaat acatgcccca 2400
   atattggaaa gacttgttct tccacggtga ctacagtaca tgctgaagcg tgccgtttca 2460
   gccctcattt aattcaattt gtaagtagcg cagcagcctc tgtgggggag gataggctga 2520
   aaaaaaaaag tgggctcgta tttatctaca ggactccata tagtcatata taggcatata 2580
   aatctattct ttttctttgt ttttttcttt cttcctttct ttcaaaggtt tgcattaact 2640
   tttcaaagta gttcctatag gggcattgag gagcttcctc attctgggaa aactgagaaa 2700
   acccatattc tcctaataca acccgtaata gcatttttgc ctgcctcgag gcagagtttc 2760
   ccgtgagcaa taaactcagc ttttttgtgg ggcacagtac tggatttgac agtgattccc 2820
   cacgtgtgtt catctgcacc caccgagcca ggcagaggcc agccctccgt ggtgcacaca 2880
   gcacgcgcct cagtccatcc cattttagtc tttaaaccct caggaagtca cagtctccgg 2940
   acaccacacc acatgagccc aacaggtcca cgatggatcc accagtccca ccccagcctt 3000
   ttcctttcat ctgaacagaa tgtgcatttt tggaagcctc cctcactctc catgctggca 3060
   gagcaggagg gagactgaag taagagatgg cagagggaga tggtggcaaa aaggtttaga 3120
   tgcaggagaa cagtaagatg gatggttccg gccagagtcg atgtggggag gaacagaggg 3180
   ctgaagggag agggggctga ctgttccatt ctagctttgg cacaaagcag cagaaagggg 3240
   gaaaagccaa tagaaatttc cttagcttcc ccaccatatg tattttctag gatttgagag 3300
   gaaagagagg aaaatggggg aatgggttgc aaaatagaaa tgagcttaat ccaggccgca 3360
   gagccaggga aggtgagtaa ctttaggagg gtgctagact ttagaagcca gataggaaga 3420
   atcagtctaa actggccatg ctttggaagg gacaagacta tgtgctccgc tgcccacctt 3480
   cagcctgcaa tgagggactg aggcccacga gtctttccag ctcttcctcc attctggcca 3540
   gtccctgcat cctccctggg gtggaggatg gaaggaaagc tgggacaagc agggaacgca 3600
   tgattcaggg atgctgtcac tcggcagcca gattccgaaa ctcccattct ccaatgactt 3660
   cctcaaccaa tgggtggcct tgtgactgtt ctttaaggct gaagatatcc aggaaagggg 3720
   gcttggacac tggccaagga gaccccttcg tgctgtggac acagctctct tcactctttg 3780
   ctcatggcat gacacagcgg agaccgcctc caacaacgaa tttggggcta cgaagaggaa 3840
   tagcgaaaaa gcaaatctgt ttcaactgat gggaacccta tagctataga acttgggggc 3900
   tatctcctat gcccctggac aggacagttg gctggggaca ggagaagtgc tcaatcttca 3960
   tgagacaaag gggcccgata gggccagcag ccacaaggcc ttgacctgcc gagtcagcat 4020
   gccccatctc tctgcacagc tgtcccctaa acccaactca cgtttctgta tgtcttaggc 4080
   cagtatccca aacctcttcc acgtcactgt tctttccacc cattctccct ttgcatcttg 4140
   agcagttatc caactaggat ctgccaagtg gatactgggg tgccactccc ctgagaaaag 4200
   actgagccag gaactacaag ctccccccac attcctccca gcctggacct aattcttgag 4260
   aggggctctc tcttcacgga ctgtgtctgg actttgagca ggcttctgcc ccttgcgttg 4320
   gctctttgct gccagccatc aggtggggga ttagagcctg gtgtaagtgc gccagactct 4380
   tccggtttcc aaagttcgtg cctgcgaacc caaacctgtg agtctcttct gcatgcagga 4440
   gtttctcctg ggcagctggt cactccccag agaagctggg ccttcatgga cacatggaac 4500
   taagcctccc aaatgggagt tctggctgag cccagggtgg ggagatcctg ggaagggagg 4560
   cactggagga agacggcacc tcttccccca tggcagggtg tgagggaggc aggtttggaa 4620
   tggtgcgagt atggcaatct aagcaggggt ctggtctctt tgactccagg ctggcctttg 4680
   gccgactgtc tgctcaccca gagaccttgg actccggact atccatggct ccgaatctaa 4740
   gtgctgccca ctcccatgct cacacccaca gaaggtcttc ccatcccctt tagattcgtg 4800
   cctcactcca ccagtgagga agatgcctct gtctttccca cgactgccag gagataggga 4860
   agcccagcca ggactgaccc tccttcctcc agcctgccct gacccacctg gcaaagcagg 4920
   gcacatgggg aggaagagac tggaaccttt ctttgacagc caggcctaga cagacaggcc 4980
   tggggacact ggccccatga ggggaggaag gcaggcgcac gaggtccagg gaggcccttt 5040
   tctgatcatg ccccttctct cccaccccat ctccccacca ccacctctgt ggcctccatg 5100
   gtacccccac agggctggcc tcccctagag ggtgggcctc aaccacctgc tcccgccacg      5160
   caccggttag tgagacaggg ctgccacggc aaccgccaag cccccctcaa ggtgggacag      5220
   taccccggac ccatccactc actcctgaga gggctccggc ccagaatggg aacctcagag      5280
   aagagctcta aggagaagaa accccatagc gtcagagagg atatgtctgg cttccaagag      5340
   aaaggaggct ccgttttgca aagtggagga gggacgaggg acaggggttt caccagccag      5400
   caacctgggc cttgtactgt ctgtgttttt aaaaccacta aagtgcaaga attacattgc      5460
   actgtttctc cactttttat tttctcttag gcttttgttt ctatttcaaa catactttct      5520
   tggttttcta atggagtata tagtttagtc atttcacaga ctctggcctc ctctcctgaa      5580
   atccttttgg atggggaaag ggaaggtggg gagggtccga ggggaagggg accccagctt      5640
   ccctgtgccc gctcacccca ctccaccagt ccccggtcgc cagccggagt ctcctctcta      5700
   ccgccactgt cacaccgtag cccacatgga tagcacagtt gtcagacaag attccttcag      5760
   attccgagtt gcctaccggt tgttttcgtt gttgttgttg ttgtttttct ttttcttttt      5820
   ttttttgaag acagcaataa ccacagtaca tattactgta gttctctata gttttacata      5880
   cattcatacc ataactctgt tctctcctct tttttgtttt caactttaaa aacaaaaata      5940
   aacgatgata atctttactg gtgaaaagga tggaaaaata aatcaacaaa tgcaaccagt      6000
   ttgtgagaaa aaaaaaaaaa agccgaaaaa aaaaaaaaaa acacctgaat gcggaagagc      6060
   tcggctcccg tttagcattt tgtacttaag gaaataaaaa accaacaaag gatctcacat      6120
   tttcttaaaa agtgaagatt gctgtatact atttattcaa cttataattt atgttactcc      6180
   ttgatctttg tcttttgtca tgacaaagca tttatttaat aaagttatgc attcagttaa      6240
   aaaaaaaaaa aaaaaaaaaa aaaaaaaaaa aaaaaaaaaa aaaaaaaaaa aaaaaaaaaa      6300
   aaaaaaaaaa aaaaaaaaaa aaaaaaaaaa aaa                                   6333
<210> 2
   <211> 272
   <212> PRT
   <213> Homo sapiens
<400> 2

## Description

The present invention relates to a new marker for radiation exposure diagnosis, and more particularly, to a marker for radiation exposure diagnosis, a composition for radiation exposure diagnosis by measuring the expression level of the marker, a kit for radiation exposure diagnosis including the composition, and a method of diagnosing radiation exposure using the marker.

The present invention is inferred from the research that has been conducted as part of nuclear research and development projects by the Ministry of Education and Science Technology [Project assignment number: 20110002359, Project Title: Development of modulation technology in radiation responses through characterization of biological signaling upon irradiation].

In a cell, which is a basic unit constituting a living body, a nucleus is contained in the center thereof, and within the nucleus, there is contained a chromosome having a regulatory role in connection with function and reproduction of the cell, wherein the chromosome has a structure of long-linked nucleic acids (e.g., DNA). When radiation separates and damages chemical bonds of the nucleic acids, the nucleic acids fail to function normally. When the nucleic acids are severely damaged, the cells lose their normal functionality, and then die. When the damage to the nucleic acids is not directly related to cell survival, the cells do not die, but changes in the nucleic acid structure have occurred therein. These changes in the cells may cause a tissue damage or a visible body change.

Radiation exposure may be caused by exposure to natural radiation, exposure to environmental pollution, or exposure to medical treatments such as chemoradiotherapy. In particular, a distribution rate of chemoradiotherapy is very high, and thus, in consideration of the current number of cancer patients, about 35% of patients in Korea and about 50% of patients in the United States are subjected to radiation therapy. In addition, the number of cancer patients receiving radiation therapy in Korea tends to increase every year.

Although radiation therapy is currently known as an essential treatment method for various types of cancer, problems have emerged in which cancer cells acquire resistance to radiation or normal tissues are damaged during radiation therapy at a high-dose rate, thereby decreasing efficiency of the treatment. In order to maximize effects of radiation therapy, the development of targeted therapeutic agents for cancer cells is required. In addition, at the same time, efforts to minimize damage to normal cells or efforts to prevent a secondary disease by tracking damage to normal cells are also needed.

Radiation exposure increases the risk of outbreaks of heart disease or cancer. In order to measure the extent of injuries to the human body damaged by radiation exposure, cytological and genetic measuring methods have been used in the past few decades. However, these measuring methods are only available to confirm whether genes or cells in the human body are damaged by radiation, and do not provide information on molecular mechanisms in the human body with respect to radiation. Thus, in order to find a marker capable of easily and accurately diagnosing radiation exposure, much effort is still being made to search for such a marker.

Over the past few decades, studies regarding markers for radiation exposure have been mainly focused on cancer. However, in recent years, the focus of the studies has expanded to discover a marker for radiation exposure that can be found in a normal tissue exposed to radiation. Such a marker may be then used for determining effects and prognosis of the human body after radiation exposure caused by nuclear accident, nuclear terrorism, or radiotherapy.

A protein is a fundamental functional unit of a cell, and has advantages as a diagnostic marker since protein can be easily collected from urine or blood, and quickly and reliably quantified in cells, body fluids, or tissues, based on the specificity of an antigen-antibody reaction, and in addition, these protein measurement methods can be automatically carried out. However, only amylase, Flt3-L, and citrulline have been proposed as a marker for radiation exposure so far. Here, amylase is known as a marker for radiation exposure in parotid glands (refer to non-patent document 1), Flt3-L is known as a marker for radiation exposure in bone marrow (refer to non-patent document 2), and citrulline is known as a marker for radiation exposure in epithelial tissues of small intestine (refer to non-patent document 3).

Organs and cells inside the body each have different sensitivities to radiation, and in this regard, cells that are less sensitive to radiation maintain their normal functions in spite of radiation exposure at lethal doses, whereas cells that are sensitive to radiation easily lose their functions or may be dead in spite of radiation exposure at very small dose. Cells that are sensitively damaged by radiation include cells with a high frequency of cell division, cells with low ploidy but high metabolic rates, and cells that are vulnerable and young. Thus, bone marrow cells and blood cells, such as white blood cells and red blood cells, are referred to as cells that are easily damaged in spite of radiation exposure even at low doses.

Cells that are subjected to organic reactions with cells in the blood and that are important for maintaining homeostasis of blood vessels are vascular endothelial cells, and in this regard, vascular endothelial cells are crucial cells since damage thereto causes vascular diseases. In addition, since the vascular endothelial cells are sensitive to radiation and changes in cells in the blood including blood cells, in the case of visible physical changes that occur due to radiation exposure, there occur vascular damage and occlusion caused by damage to the vascular endothelial cells. Therefore, it is very important to contrive a method of suppressing damage due to radiation exposure while measuring the extent of radiation exposure of the vascular endothelial cells at the same time.

### [Documents of related arts]

### [Non-patent documents]

1. Van den Brenk et al., Serum amylase as a measure of salivary gland radiation damage. Hyperamylasaemia following fractionated exposure to 4 MV X rays delivered in high pressure oxygen, and effects of certain steroids on this response. Br J Radiol 1969, 42, 688-700.
2. Siegel, J. A. et al., Red marrow radiation dose adjustment using plasma FLT3-L cytokine levels: improved correlations between hematologic toxicity and bone marrow dose for radioimmunotherapy patients. J Nucl Med 2003, 44, 67-76.
3. Lutgens, L. C. et al., Citrulline: a physiologic marker enabling quantitation and monitoring of epithelial radiation-induced small bowel damage, Int J Radiat Oncol Biol Phys 2003, 57, 1067-1074.

### DETAILED DESCRIPTION OF THE INVENTION

The invention is as defined in the accompanying claims.

In this regard, inventors of the present invention have completed the present invention as a result of research to develop a protein or its genetic marker of which the expression level varies in vascular endothelial cells upon radiation exposure or irradiation, thereby finding out that particular genes in the vascular endothelial cells can act as markers for radiation exposure in connection with cell damage caused by radiation exposure.

Therefore, one objective of the present invention is to provide a new genetic marker or its protein marker with respect to radiation exposure.

Disclosed herein but not claimed is a composition for radiation exposure diagnosis including an agent for measuring an expression level of the genetic marker at an mRNA or protein level.

Disclosed herein but not claimed is a kit for radiation exposure diagnosis including the composition.

An objective of the present invention is to provide a method of diagnosing radiation exposure by comparing expression levels of the genetic marker at an mRNA or protein level upon radiation exposure with those of a normal control group.

Another objective of the present invention is to provide a method of screening an agent for enhancing radiation sensitivity or an agent for radiation protection, the method including selecting a sample having increased or decreased expression levels of the genetic marker at an mRNA or protein level upon radiation exposure compared to the expression levels of a control group.

Disclosed herein but not claimed is a composition for enhancing radiation sensitivity or a composition for radiation protection in which an agonist or an antagonist to the expression or action of the genetic marker at an mRNA or protein level upon radiation exposure is included.

Disclosed herein but not claimed is a composition for radiation exposure diagnosis including an agent for measuring expression levels of an insulin-like growth factor binding protein 5 (IGFBP-5) gene at an mRNA or the protein.

Disclosed herein but not claimed is a kit for radiation exposure diagnosis including the composition.

An aspect of the present invention provides a method of diagnosing radiation exposure, the method including:
measuring expression levels of an *IGFBP-5* gene at an mRNA or the protein in a patient's sample that is obtained from vascular endothelial cells;
comparing the measured expression levels of the *IGFBP-5* gene at an mRNA or the protein with those of a normal control group; and
determining the patient to be exposed to radiation in the case of higher expression levels in the patient's sample than those in the normal control group.

Another aspect of the prevent invention provides a method of screening an agent for enhancing radiation sensitivity, the method including:
treating cells of a vascular endothelial cell line with individual samples of the agent;
measuring expression levels of an *IGFBP-5* gene at an mRNA or the protein in the vascular endothelial cells; and
selecting a sample of the agent having increased expression levels of the *IGFBP-5* gene at an mRNA or the protein compared to those of a control group.

Another aspect of the present invention provides a method of screening an agent for radiation protection, the method including:
treating cells of a vascular endothelial cell line with individual samples of the agent;
measuring expression levels of an *IGFBP-5* gene at an mRNA or the protein in the vascular endothelial cells; and
selecting a sample of the agent having decreased expression levels of the *IGFBP-5* gene at an mRNA or the protein compared to those of a control group.

Disclosed herein but not claimed is a composition for enhancing radiation sensitivity including an agonist to expression or action of an *IGFBP-5* gene at an mRNA or the protein.

Disclosed herein but not claimed is a composition for radiation protection including an antagonist to expression or action of an *IGFBP-5* gene at an mRNA or the protein.

Hereinafter, the present invention will be described in more detail.

All the technical terminologies used in the present invention, unless otherwise defined, will be understood by those of ordinary skill in the art.

The present invention, as a result of intensive studies into finding a genetic marker or its protein marker for radiation exposure, has been designed for use of the IGFBP5 as a marker for radiation exposure, based on the finding that the expression levels of IGFBP5 and the expression levels of the *IGFBP5* gene at an mRNA level are reduced upon radiation exposure of the vascular endothelial cells.

According to an embodiment of the present invention, the expression level of IGFBP5 is significantly changed after human vascular endothelial cells (HUVECs) that have high sensitivity to radiation are irradiated, and thus, it is confirmed that IGFBP5 has an important role in causing damage to the cells due to radiation. As a result, IGFBP5 may be applicable as a biomarker for radiation exposure, and at the same time, a drug for controlling radiation damage may be developed using the expression level of IGFBP5 gene as a target. In detail, after irradiation to HUVECs, it is first found that the expression levels of IGFBP5 and the expression levels of the *IGFBP5* gene at an mRNA level among a great number of proteins that can be identified by a proteomics method are significantly increased compared to those of a normal control group (see Examples 1 to 3). Here, in consideration of the radiation marker IGFBP5 in the HUVECs, it is confirmed that apoptosis due to radiation is promoted when the radiation marker IGFBP5 is overexpressed in the HUVECs (see Example 4), whereas apoptosis due to radiation is decreased when knockdown of the radiation marker IGFBP5 occurs. In addition, degradation of angiogenesis due to radiation is recovered when knockdown of the radiation marker IGFBP5 occurs (see Example 5).

Therefore, in some embodiments of the present invention, provided is use of IGFBP5 as a marker for radiation exposure diagnosis.

The term "diagnosis" typically refers to identification of pathophysiological status or features, and for the purpose of the present invention, the term refers to whether a subject is exposed to radiation and/or refers to the extent of the radiation exposure.

The term "diagnostic marker", "marker for diagnosis", or "diagnosis marker" used herein refers to a material which is capable of diagnosing between an experimental group and a normal control group by comparing samples of each group. Examples of the material include organic biomolecules such as polypeptides or nucleic acids (e.g., mRNA), lipids, glycolipids, glycoproteins, or sugars (e.g., monosaccharide, disaccharaide, oligosaccharides, etc), wherein the expression level of the diagnostic marker is either increased or decreased in a radiation-exposed group compared to the normal control group. The marker for radiation exposure diagnosis used herein is mRNA of the *IGFBP5* gene or a protein encoded by the *IGFBP5* gene, wherein the *IGFBP5* gene is highly expressed particularly in vascular endothelial cells in the radiation-exposed group, compared to vascular endothelial cells in the normal group.

Human IGFBP5 is insulin-like growth factor binding protein 5, and information on genes or proteins of human IGFBP5 has been registered in the National Center for Biotechnical Information (NCBI) [GeneBank Accession No.: NM_000599(SEQ ID NO: 1), CAG33090(SEQ ID NO: 2)].

Radiation exposure and extent of the exposure may be diagnosed by measuring the expression levels of the IGFBP5 marker at an mRNA or the protein.

Disclosed herein but not claimed is a composition for radiation exposure diagnosis including an agent for measuring the expression levels of the *IGFBP5* gene at an mRNA or the protein.

The term "an agent capable of measuring the expression levels of *IGFBP5* gene at an mRNA or the protein" used herein refers to a molecule that can be used in detection and/or quantification of the IGFBP5 marker by identifying the expression levels of the *IGFBP5* gene at an mRNA or the protein. The agent may be a primer or a probe that binds specifically to the *IGFBP5* gene, an antibody specific to IGFBP5, or a partial peptide having a binding domain specific to IGFBP5.

The agent for measuring the mRNA expression level of the *IGFBP5* gene may be a primer or a probe that binds specifically to mRNA of the *IGFBP5* gene. In this regard, since nucleic acid sequences in mRNA of the *IGFBP5* genes are known, one of ordinary skill in the art may design a primer or a probe that binds specifically to mRNA of the *IGFBP5* gene based on the known sequences.

The term "measurement of the mRNA expression level" used herein refers to a process of determining the presence of mRNA expression and extent of the mRNA expression of a gene for radiation exposure diagnosis in a biological sample, thereby diagnosing radiation exposure or extent of the irradiation. In this regard, the quantity of mRNA is measured, and in some embodiments, the quantity of mRNA may be measured using a primer or a probe for the mRNA. Examples of the assay methods available for measuring the mRNA expression level include RT-PCR, competitive RT-PCR, real-time RT-PCR, RNase protection assay (RPA), and Northern blotting, or a method using a DNA microarray chip, but are not limited thereto.

According to the detection methods of the related art, the mRNA expression level in the normal control group may be compared with that of the biological sample obtained from a subject. In addition, based on the determination of significant changes in the mRNA expression level in the *IGFBP5* gene, radiation exposure or extent of the irradiation may be determined. The measurement of the mRNA expression level may be achieved preferably using RT-PCR with primers specific to the *IGFBP5* gene. RT-PCR is a method that has been introduced by P. Seeburg (Cold Spring Harb Symp Quant Biol 1986, Pt 1:669-677) to analyze RNA, and in this regard, cDNA obtained by reverse transcription of mRNA is amplified by PCR for analysis. Here, in the amplification step, pairs of primers manufactured to be specific to the *IGFBP5* gene are used. After RT-PCR, expression of mRNA of the *IGFBP5* gene and expression level thereof may be determined by examining the patterns and thicknesses of the bands separated upon electrophoresis. In addition, the mRNA expression level of the *IGFBP5* gene may be compared with that of the normal control group so as to simply diagnose radiation exposure or extent of the irradiation.

The term "primer" used herein refers to a short strand of nucleic acid sequence which can form base pairings with a complementary template and has a free 3'-hydroxyl group serving as a starting point for replication of template strands. DNA synthesis can start with primers in the presence of a polymerase reagent (e.g., DNA polymerase or reverse transcriptase) under the proper conditions of buffer solutions and temperatures, and 4 different nucleoside triphosphates. In an embodiment of the present invention, a marker gene is amplified by PCR using a set of sense and antisense primers that are specific to mRNA of the *IGFBP5* gene and have 7 to 50 nucleotide sequences, so as to diagnose radiation exposure by measuring yields of desirable product. Here, the set of sense and antisense primers may further include additional features so long as the basic properties of the primers acting as a starting point of DNA synthesis are not changed. PCR conditions and lengths of sense and antisense primers may be appropriately selected according to techniques known in the art.

The term "probe" used herein refers to a nucleic acid fragment, such as a DNA or RNA fragment, formed of from short a few bases to long several hundred bases. The probe may be labeled to determine the presence or absence of a target mRNA and quantity (expression level) of the target mRNA. The probe may be manufactured in the form of an oligonucleotide probe, a single-stranded DNA probe, a double-stranded DNA probe, or an RNA probe. In an embodiment of the present invention, hybridization between mRNA of an IGFBP5 polynucleotide and a complementary probe allows the diagnosis of radiation exposure and extent of the irradiation by measuring the mRNA expression level in the hybridization. Choice of suitable probes and hybridization conditions may be appropriately selected according to techniques known in the art.

The primers or probes of the present invention may be chemically synthesized using a phosphoramidite solid-phase method or another well-known method. In addition, these nucleic acid sequences (i.e., primers or probes) may be modified using methods known in the art. Examples of the modification are methylation, capping, substitution with at least one analogue of natural nucleotides, and internucleosidic modification, for example, modification with non-charged linkers (e.g., methylphosphonate, phosphotriester, phosphoroamidate, carbamate, etc) or charged linkers (e.g., phosphorothioate, phosphorodithioate, etc). The primers or probes of the present invention may be also modulated using a label that can directly or indirectly provide a detectable signal. Examples of the label are radioisotopes, fluorescent molecules, and biotin.

According to an embodiment, the agent for measuring the protein expression level is an antibody that binds specifically to the protein, or a partial peptide having a binding domain that is specific to the protein.

The term "measurement of the protein expression level" used herein refers to a process of determining the presence of protein expression and extent of the protein expression of a gene for radiation exposure diagnosis in a biological sample, thereby diagnosing radiation exposure or extent of the irradiation. In an embodiment, the proteins may be confirmed using an antibody that binds specifically to a protein, or a partial peptide having a binding domain that is specific to a protein. Examples of the assay methods available for measuring the protein expression level include Western blotting, enzyme linked immunosorbent assay (ELISA), radioimmunoassay (RIA), radioimmunodiffusion, Ouchterlony immunodiffusion, rocket immunoelectrophoresis, histoimmunostaining, immunoprecipitation assay, complement fixation assay, and FACS, or a method using a protein chip, but are not limited thereto.

The measurement of the protein expression level may be achieved preferably using ELISA. Various types of ELISAs include direct ELISA in which a labeled antibody immobilized onto a solid support is used to recognize an antigen, indirect ELISA in which a labeled antibody is used to recognize a captured antibody immobilized on a solid support which is complex with an antigen, direct sandwich ELISA in which an antibody is used to recognize an antigen captured by another antibody immobilized onto a solid support, and indirect sandwich ELISA in which a secondary antibody is used to recognize an antibody which captures an antigen complexed with a different antibody immobilized onto a solid support. More preferably, an antibody is immobilized onto a solid support and is reacted with a sample to form an antigen-antibody complex, and then, a labeled antibody specific to the antigen of the antigen-antibody complex is allowed to capture the antigen of the complex, followed by enzymatic color development. Alternatively, an antibody specific to the antigen of the antigen-antibody complex is allowed to capture the antigen of the complex and then is recognized by a labeled secondary antibody, followed by enzymatic color development. The formation of the complex of a marker protein with an antibody may be quantitatively measured so as to diagnose radiation exposure or extent of the irradiation.

In addition, in some embodiments, the measurement of the protein expression level may be achieved preferably using Western blotting with at least one antibody for the proteins. The entire proteins are selected from a sample, separated according to size by electrophoresis, transferred onto a nitrocellulose membrane, and reacted with an antibody to form an antigen-antibody complex. The expression level of the protein is measured by determining the quantity of the complex using a labeled antibody, thereby confirming radiation exposure or extent of the irradiation.

The detection method may be conducted by measuring the protein expression level in the normal control group and the biological sample. The expression levels of mRNA or protein may be represented by differences between absolute (e.g., *µ*g/mℓ) or relative (e.g., relative intensity of signal) scales of the marker.

The term "antibody" as a term known in the art refers to a specialized immunoglobulin which is directed toward an epitope. The antibody used in the present invention refers to an antibody that binds specifically to IGFBP5, which is a marker for radiation exposure of the present invention. The antibody may be prepared using IGFBP5. Here, the *IGFBP5* gene is cloned in an expression vector so as to obtain a protein, i.e., IGFBP5, encoded by the *IGFBP5* gene. The antibody may be in the form of a polyclonal antibody, a monoclonal antibody, and a recombinant antibody, and in this regard, all the immunoglobulin antibodies fall within the range of the antibody of the present invention. The antibody is in a complete form composed of two full-length light chains and two full-length heavy chains. In addition, special antibodies such as humanized antibodies are among the antibodies of the present invention. The antibodies may be used to confirm expression of the protein in a biological sample according to the known assays in the art, such as ELIZA, RIA, sandwich assay, Western blotting or Immunoblotting for a polyacryl gel.

The term "partial peptide having a binding domain specific to a protein" used herein refers to a polypeptide that does not have a complete antibody structure, but has an antigen-binding site (i.e., a binding domain) which is directed toward an epitope. The partial peptide includes functional fragments of antibody molecules rather than intact antibodies composed of two light chains and two heavy chains. The functional fragments of antibody molecules mean fragments retaining at least antigen-binding functionality, and examples thereof include Fab, F(ab'), F(ab')₂, and Fv. The partial peptide includes at least 7 amino acids, preferably at least 9 amino acids, and more preferably, at least 12 amino acids.

The expression levels of the *IGFBP5* gene at an mRNA level and the protein are increased in vascular endothelial cells upon radiation exposure, and in this regard, the composition for detecting a marker for radiation exposure diagnosis of the present invention may be used to measure protein expression levels in a sample obtained from vascular endothelial cells among subjects suspected of radiation exposure.

Disclosed herein but not claimed is a kit for diagnosis of radiation exposure, the kit including the composition for diagnosis of radiation exposure.

The kit may diagnose radiation exposure by measuring the expression levels of *IGFBP5* genes of the radiation exposure marker at an mRNA or the protein. The kit includes the agent such as primers or probes for measuring the above-described *IGFBP5* gene at an mRNA or the protein, i.e., primers or probes binding specifically to mRNA of the *IGFBP5* gene, antibodies binding specifically to IGFBP5, partial peptides having a binding domain that binds specifically to IGFBP5, or a combination thereof. In addition, the kit may include one or more components, solutions, or devices suitable for an analysis method to measure the expression level of IGFBP5.

The kit for radiation exposure diagnosis may be a microarray for radiation exposure diagnosis that can measure expression levels of protein or mRNA of the protein-coding gene. The microarray for radiation exposure diagnosis may be easily manufactured by one of ordinary skill in the art according to methods known in the art using the marker of the present invention. The microarray may include mRNA of the IGFBP5-coding gene or cDNA with sequence corresponding to a fragment of the marker gene as a probe attached to a substrate.

When the kit for radiation exposure diagnosis is used to measure expression levels of the *IGFBP5* gene at an mRNA level, the kit may include essential elements necessary for RT-PCR. The RT-PCR kit may include test tubes or other suitable containers, reaction buffers, deoxyribonucleotides (dNTPs), enzymes such as Taq-polymerase and reverse transcriptase, a deoxyribonuclease (DNase), RNase inhibitor, dEPC-water, sterile water, and so on, in addition to primers each of which is specific to mRNA of the marker genes. Also, the kit may include a pair of primers specific to genes that are used as a quantitative control group.

The kit may include antibodies binding specifically to IGFBP5, substrates for immunological detection of antibodies, suitable buffer solutions, coloring enzymes or fluorescent-labeled secondary antibodies, or coloring substrates. The matrix may be a nitrocellulose membrane, a 96-well plate made of polyvinyl resin or polystyrene resin, a glass slide glass. The coloring enzyme may be peroxidase or alkaline phosphatase, and the fluorescent material may be FITC or RITC. The coloring substrate may be 2,2'-azino-bis(3-ethylbenzothiazoline-6-sulfonic acid (ABTS), o-phenylenediamine (OPD), or tetramethyl benzidine (TMB).

Another aspect of the present invention provides a method of diagnosing radiation exposure, the method including:
measuring expression levels of an *IGFBP-5* gene at an mRNA or the protein in a sample from a patient, wherein the sample is obtained from vascular endothelial cells;
comparing the measured expression levels of the *IGFBP-5* gene at an mRNA or the protein with that of a normal control group; and
determining the patient to be exposed to radiation when the expression levels of the patient's sample are higher than those of the normal control group.

The term "patient" used herein refers to a subject suspected of any exposure to radiation such as exposure to natural radiation, exposure to pollution, or anticancer treatment. The patient includes any subject of which the expression levels of the *IGBP5* gene at an mRNA or the protein are changed due to any exposure to radiation.

The term "sample from a patient" used herein refers to tissues, cells, whole blood, sera, plasma, sputum, saliva, cerebrospinal fluid or urine, in which the radiation exposure marker IGFBP5 shows different expression levels thereof before and after radiation exposure. Examples of the sample from the patient are not limited thereto, and preferably, the sample from the patient may be a sample obtained from vascular endothelial cells.

According to the diagnostic method described above, information necessary for diagnosis of radiation exposure may be provided. In detail, the diagnostic method includes detecting expression level of the *IGFBP5* gene at an mRNA or protein level by separating protein or mRNA from a patient's sample, wherein the separating of protein or mRNA of the *IGFBP5* gene may be carried out appropriately by one or ordinary skill in the art according to methods known in the art. According to an embodiment, after vascular endothelial tissues of the patient are homogenized with a protein extraction buffer or nucleic acid extraction buffer, supernatant obtained by centrifugation may be may used as a sample of the patient.

According to another aspect of the present invention, provided is a method of screening an agent for enhancing radiation sensitivity, the method comprising:
treating cells of a vascular endothelial cell line with individual samples of the agent;
measuring expression levels of an *IGFEB-5* gene at an mRNA or the protein in the vascular endothelial cells; and
selecting a sample of the agent having increased expression levels of the *IGFBP-5* gene at an mRNA or the protein compared to those of a control group.

According to another aspect of the present invention, provided is a method of screening an agent for radiation protection, the method comprising:
treating cells of a vascular endothelial cell lines with individual samples of the agent;
measuring expression levels of an *IGFBP-5* gene at an mRNA or the protein in the vascular endothelial cell lines; and
selecting a sample of the agent having decreased expression levels of the *IGFBP-5* gene at an mRNA or the protein compared to those of a control group.

It is confirmed that when the radiation marker IGFBP5 in HUVECs is overexpressed, cell death caused by radiation is accelerated (Example 4), and when knockdown of the radiation marker IGFBP5 in HUVECs occurs, cell death caused by radiation is decreased and angiogenesis that has been degraded by radiation is then recovered (Example 5). Accordingly, it is confirmed that when the expression level of IGFBP-5 is increased, the action of cells against radiation becomes sensitive, and when the expression level of IGFBP-5 is decreased, the damage to cells due to radiation is reduced. Therefore, when the expression levels of the *IGFBP-5* gene at an mRNA or the protein are increased compared to those of a control group after treating vascular endothelial cell lines with individual samples, the sample may be said be effective as a sensitizer for radiation. On the other hand, when the expression levels of the *IGFBP-5* gene at an mRNA or the protein are decreased compared to those of a control group, the sample may be said to be effective as an agent for radiation protection.

In consideration of the methods of diagnosing radiation exposure and the method of screening the agent for enhancing radiation sensitivity and the agent for radiation protection, one of ordinary skill in the art may appropriately select a method of measuring the expression levels of the *IGFBP-5* gene at an mRNA or the protein from the methods of the present invention in connection with the above-described composition and kit for radiation exposure diagnosis.

Disclosed herein but not claimed is a composition for enhancing radiation sensitivity including an agonist to expression or action of the *IGFBP-5* gene at an mRNA or the protein.

Disclosed herein but not claimed is a composition for radiation protection including an antagonist to expression or action of the *IGFBP-5* gene at an mRNA or the protein.

Since the overexpression of the radiation marker IGFBP5 in vascular endothelial cells accelerates cell death caused by radiation (Example 4), a material increasing or accelerating expression or action of the *IGFBP5* gene at an mRNA or the protein, i.e., an agonist to expression or action of the *IGFBP-5* gene at an mRNA or the protein, may act as an agent for enhancing radiation sensitivity to enhance radiation action. In addition, since it is confirmed that the knockdown of the radiation marker IGFBP5 in vascular endothelial cells (decreases cell death caused by radiation and recovers angiogenesis that has been degraded by radiation (Example 5), a material decreasing or inhibiting expression or action of the *IGFBP5* gene at an mRNA or the protein, i.e., an antagonist to expression or action of the *IGFBP-5* gene at an mRNA or protein level, may act as an agent for radiation protection that can inhibit radiation action on the living body to inhibit damage caused by radiation.

The term "radiation protection" used herein refers to practice of inhibiting radiation damage to tissues or cells of the living body by blocking or weakening radiation damage to tissues or cells of the living body.

The composition for enhancing radiation sensitivity can enhance sensitivity of the living body to radiation action, and thus may further enhance effects thereof in the treatment of cancer by radiation. The composition for radiation protection may control radiation action on normal tissues or cells when the normal tissues are irradiated. The normal tissues or cells may be any tissue or cell in which IGFBP5 exists, and for example, the normal tissues or cells may be vascular endothelial tissues or vascular endothelial cells.

The antagonist may be any one selected from antisense nucleic acid or small interfering RNA which is capable of complementarily binding with mRNA of the gene. In addition, the antagonist may be at least one selected from the group consisting of compounds, peptides, and antibodies that bind complementarily to the protein.

The antisense nucleic acid may be introduced into cells to enhance sensitivity to radiation. The term "introduction" used herein refers to introduction of foreign DNA into cells by transfection or transduction. The transfection may be performed by various methods known in the art, such as calcium phosphate-DNA co-precipitation, DEAE-dextran mediated transfection, polyberene-mediated transfection, electroporation, microinjection, ribosome fusion method, lipofectamine and electroporation. The transduction is a method of delivering a gene into cells using virus or virus vector particles by means of infection.

The term "inhibition of damage caused by radiation" used herein refers to a practice of controlling damage to normal cells against radiation, in consideration of biological responses to radiation including radiation treatment. Accordingly, damage to tissues and cells of the living body against radiation exposure may be inhibited, and thus the living body may be irradiated with sufficient radiation for radiotherapy. In addition, at the same time, the efficiency of the radiotherapy may be increased.

The agonist or antagonist to expression or action of the *IGFB-5* gene at an mRNA or the protein each included in the composition for enhancing radiation sensitivity or in the composition for radiation protection may be a sufficient amount for expression or inhibition of the expression. Such an amount and daily doses thereof may be determined by one of ordinary skill in the art according to conventional methods known in the art. These doses may vary according to weight, gender, race, age, and illness of the target subject, and accordingly may be added or subtracted by determination of a specialized doctor.

The compositions for diagnosis of radiation exposure, for enhancement of radiation sensitivity, and for protection from radiation may further include a pharmaceutically acceptable carrier, and then, may be formulated with the carrier.

The term "pharmaceutically acceptable carrier" used herein refers to a carrier or a diluent that does not stimulate an organism nor hinder biological activities or characteristics of compounds to add. A pharmaceutical carrier that is acceptable in regard to a composition formulated as a liquid solution may be sterile and biocompatible, and may be used by mixing saline solution, sterile water, Ringer's solution, buffered saline, albumin injection solution, dextrose solution, maltodextrin solution, glycerol, ethanol with at least one component of the above examples, and other conventional additives such as antioxidants, buffers, and fungistatic agents may be further added if necessary. In addition, diluents, dispersants, surfactants, binders, and lubricants may be further added so as to formulate the composition as an injectable formulation including aqueous solutions, suspensions, and emulsions, pills, capsules, granules or tablets.

The compositions for enhancing radiation sensitivity and for radiation protection may be applicable in any formulation, and in this regard, the composition may be prepared for oral formulation or parenteral formulation. In addition, in consideration of ease of administration and uniformity of dose, the compositions may be formulated in unit dosage forms. The pharmaceutical formulation may be in a suitable form for oral, rectal, nasal, topical (including cheek and beneath a tongue), subcutaneous, vaginal, or parenteral (including intramuscular, subcutaneous, and intravenous) administration, or may be in a suitable form for inhalation or insufflation.

The oral dosage formulation may be in the form of, for example, tablets, troches, lozenge, soluble or oil suspensions, powders or granules, emulsions, hard or soft capsules, syrups, or elixirs. In order to prepare the formulation in the form of tablets and capsules, the formulation may further include binding agents such as lactose, saccharose, sorbitol, mannitol, starch, amylopectin, cellulose, or gelatin; excipients such as dicalcium phosphate, and disintegrating agents such as corn starch or sweet potato starch; and lubricants such as stearate magnesium, stearate calcium, sodium stearyl fumarate, or polyethyleneglycol wax. In the case of the capsule-type formulation, the formulation may further include a liquid carrier such as fatty oil, in addition to the above-described materials.

In addition, the formulation for parenteral administration may be in an injectable form including subcutaneous injection, intravenous injection, or intramuscular injection, in a suppository form, or in a spray form such as an aerosol that enables inhalation through the respiratory tract. In order to prepare the formulation in an injectable form, the composition is mixed with a stabilizer or buffer in water so as to prepare a mixed solution or suspension. Then, the mixed solution or suspension may be formulated in an ampule or vial unit for administration. For administration in the form of a suppository, the formulation may be prepared as the composition for rectal administration, such as a suppository or a physical therapy enema including typical suppository bases such as cocoa butter and other glycerides. In the case of formulation in a spray form such as an aerosol, propellants used to disperse waterborne concentrates or damp powder may be blended with additives.

According to the present invention, based on the finding of a new IGFBP-5 marker for diagnosis of radiation exposure in which an expression level of the marker is increased due to radiation exposure, the radiation exposure and an extent thereof may be determined by using a composition or a kit for diagnosis of radiation exposure, wherein the composition includes an agent for measuring the expression level of the marker IGFBP-5 gene at an mRNA or the protein, and the kit includes the composition. In addition, the selecting of a material that increases the expression levels of the *IGFBP-5* gene at an mRNA or the protein may lead to screening of an agent for enhancing radiation sensitivity, whereas the selecting of a material that decreases the expression levels of the *IGFBP-5* gene at an mRNA or the protein may lead to screening of an agent for radiation protection. In addition, an agonist to expression or action of the *IGFBP-5* gene at mRNA or the protein may be used as an agent for enhancing radiation sensitivity, and an antagonist to expression or action of the *IGFBP-5* gene at mRNA or the protein may be used as an agent for radiation protection.

### DESCRIPTION OF THE DRAWINGS

FIG. 1 is a graph showing types and contents of genes obtained as a result of microarrays assay measuring the intracellular genes after irradiation of human umbilical vein endothelial cells (HUVECs).
FIG. 2 is a table describing upregulated genes and down-regulated genes among genes obtained as a result of microarrays assay measuring the intracellular genes after irradiation of HUVECs.
FIG. 3A is an image showing a result of quantified amounts of mRNA of *IGFBP5* genes by glyceraldehyde 3-phosphate dehydrogenase (GAPDH), wherein the mRNA is extracted from HUVECs after irradiation.
FIG. 3B is a graph showing a result of quantified amounts of mRNA of *IGFBP5* genes by GAPDH, wherein the mRNA is extracted from HUVECs after irradiation.
FIG. 3C is an image showing amounts of IGFBP5 protein determined by Western blotting, wherein the IGFBP5 protein is extracted from HUVECs after irradiation.
FIG. 4A is a graph showing a result of viability of cells that overexpress IGFBP5 by infection of IGFBP5 or do not overexpress IGFBP5 measured according to MTT analysis, wherein the cells are either irradiated or not irradiated with 4 Gy of radiation.
FIG. 4B is an image showing content of various proteins including IGFBP5 determined by Western blotting after extracting proteins from cells that overexpress IGFBP5 by infection of IGFBP5 or do not overexpress IGFBP5, wherein the cells are either irradiated or not irradiated with 4 Gy of radiation.
FIG. 5A is a graph illustrating a result of viability of cells measured according to MTT analysis, wherein the cells are either irradiated or not irradiated with 4 Gy of radiation after knockdown of the IGFBP5 occurs due to IGFBP5 miRNA infection.
FIG. 5B is an image showing content of various proteins including IGFBP5 determined by Western blotting after extracting proteins from cells, wherein the cells are either irradiated or not irradiated with 4 Gy of radiation after knockdown of the IGFBP5 occurs due to IGFBP5 miRNA infection.
FIG. 6A shows images illustrating angiogenic status in the case of irradiation with 4 Gy of radiation to the cells in which IGFBP5 knockdown occurs due to IGFBP5 miRNA infection.
FIG. 6B is a graph showing a result of measuring length of blood vessel formed by irradiating with 4 Gy of radiation to cells in which IGFBP5 knockdown occurs due to miRNA infection in comparison with those of a control group.

### <Description of drawing symbols>

v: No IGFBP5 overexpression
IGFBP5: IGFBP5 overexpression
IR: Irradiation
NC: negative control
miR-BP5: IGFBP5 miRNA infection group

Hereinafter, the present invention will be described in more detail with reference to exemplary embodiments described below.

### <Example 1> Cell line incubation and irradiation thereto

Human umbilical vein endothelial cells (HUVECs) were added with 2 wt% FBS and several growth factors in endothelial cell basal medium-2(EBM-2), and then incubated in an incubator under 37°C with 5 wt% Co₂. Irradiation was carried out using gamma rays, i.e., caesium-137 gamma ray sources (¹³⁷Cs γ-ray, Atomic Energy of Canada, Ltd, Canada). The growing cells were irradiated by 4 Gy γ-rays (for 1 minutes and 25 seconds) at a rate of 2.82 Gy/min, and then used in experiments.

### <Example 2> Discovery of radiation exposure diagnostic gene by microarray analysis

### 1. RNA extraction

According to the manufacturer's instructions, 1 ml TRIzol (Invitrogen, Carlsbad, California) was added to a plate on which the cultured and irradiated cell lines of Example 1 were dispensed, and then, 0.2 ml of chloroform was added thereto so as to extract mRNA. The sample was shaken for 15 to 20 seconds, incubated at room temperature for 2 to 3 minutes, treated with ice-cold isopropanol to separate an aqueous layer therefrom, and washed in ice-cold 70% ethanol. RNA pellets were then air-dried to be resuspended in diethylprocarbonate (DEPC)-treated water, and a small amount of the sample was extracted for the purpose of agarose gel analysis and spectrometric quantitative analysis.

### 2. Microarray analysis

Each total RNA (10 µg) sample that was previously prepared was labelled with Cy5-conjugated dCTP (Amersharm, Piscataway, NJ) using SuperScrip II reverse transcriptase (Invitrogen, Carlsbad, California). The labelled cDNAs were concentrated by ethanol precipitation, and were placed on a Roche NimbleGen Human whole genome 12-plex array (Roche NimbleGen, Inc., WI) and covered by a NimbleGen H12 mixer (Roche NimbleGen, Inc., WI). Slides were used for a MAUI system (Biomicro systems, Inc. UT) hybridization reaction at a temperature of 42°C for 12 hours. These hybridized slides were washed in 2X SSC at room temperature, in 0.1 wt% SDS for 2 minutes, in 1 X SSC for 3 minutes, and then, in 0.2 X SSC for 2 minutes. The slides obtained therefrom were then dried by centrifugation at a speed of 3,000 rpm for 20 seconds.

### 3. Data analysis

The arrays were submitted to Roche NimbleGen Inc., wherein the arrays had twelve copies of each genome per chip therein. It was found that three different 60-based oligonucleotides (60-mer probes) in average were each gene of the genome. In this regard, by comparing all the other 60-mer probes in a target genome, probes each including at least 3 mismatches were selected. A practice of checking (i.e., hybridization) the control group was performed in each array including on-chip control oligonucleotides. These arrays were then analyzed using an Axon GenePix 4000B scanner having associated software. Gene expression levels were calculated with NimbeScan Version 2.4 (Roche NimbleGen, Inc., WI). Relative signal intensities for each gene were calculated using a Robust Multi-Array Average algorithm. Each of the data was processed based on a median polish normalization method using NimbeScan Version 2.4 (Roche NimbleGen, Inc., WI). The normalized and log transformed intensity values were then analyzed using GeneSpring GX 10 (Agilent technologies, CA). Fold change filters included the requirement that the genes be present in at least 200% of controls for up-regulated genes, and in lower than 50% of controls for down-regulated genes.

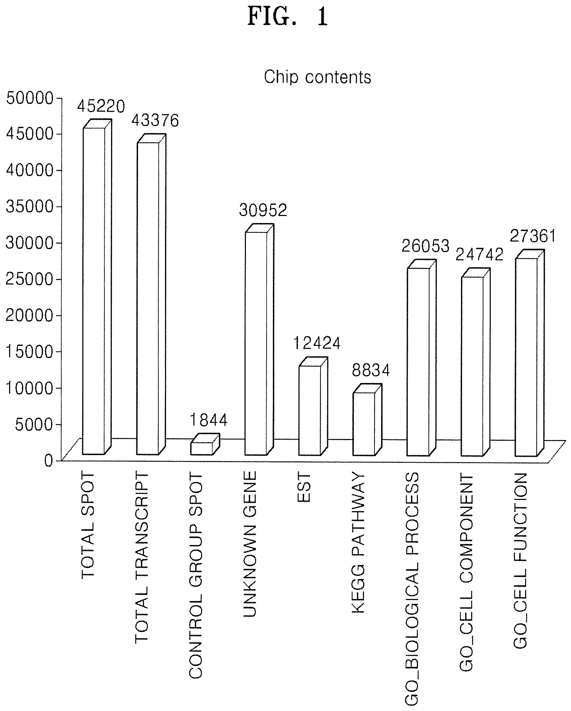

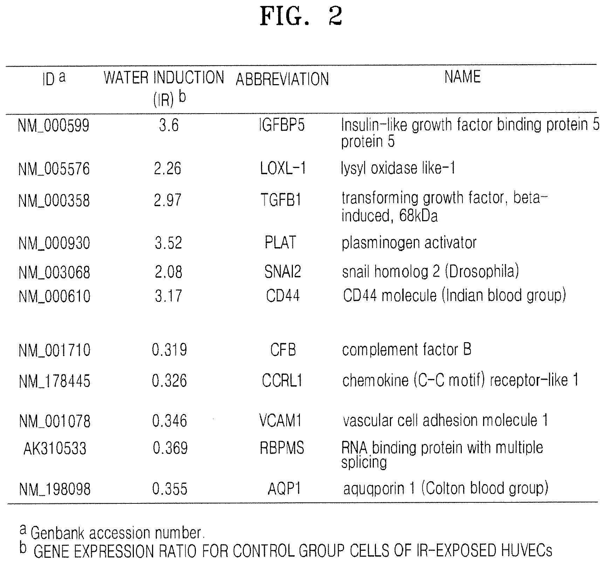

The classification of the genes obtained as a result is shown in FIG. 1, and the genes that are changeable based on the presence or absence of radiation are listed in a table as shown in FIG. 2.

FIG. 1 is a graph showing types and contents of genes obtained as a result of microarrays assay measuring the intracellular genes after irradiation of HUVECs.

FIG. 2 is a table describing upregulated genes and down-regulated genes among genes obtained as a result of microarray assays measuring the intracellular genes after irradiation of HUVECs.

### <Example 3> Confirmation of expression of genes discovered

For the purpose of re-confirmation of microarray data of Example 2, RT-PCR and real-time PCR were performed.

### 1. RT-PCR analysis

2 ug of the extracted and quantified RNA sample had oligo dT and dNTP added thereto, and was then heated at a temperature of 65°C for 5 minutes, followed by being cooled on ice. Here, 5X First strand buffer (Invitrogen superscript II kit), 0.1M DTT, and RNase inhibitor were added to the sample. Following heating at a temperature of 42°C for 2 minutes, 1 ul of superscript II was added to the sample, and then the sample was allowed to stand at a temperature of 42°C for 1 hour. Then, the sample was heated at a temperature of 70°C for 15 minutes, thereby completing the reaction to synthesize cDNAs. These synthesized cDNAs were used to perform PCR at an appropriate annealing temperature for each primer.

### 2. Real-time PCR analysis

RNA was extracted and quantified so as to perform real-time PCR using One Step SYBR PrimeScript™ RT-PCR Kit (Takara, Osaka, Japan) and DNA Engine2.OPTICON (MJ Reserch, MA, USA). The gene expression was quantified by glyceraldehyde 3-phosphate dehydrogenase (GAPDH).

### 3. Measurement of protein expression

After proteins were extracted from HUVECs that were irradiated or HUVECs that were not irradiated, levels of IGFBP5 expression were measured using Western blotting.

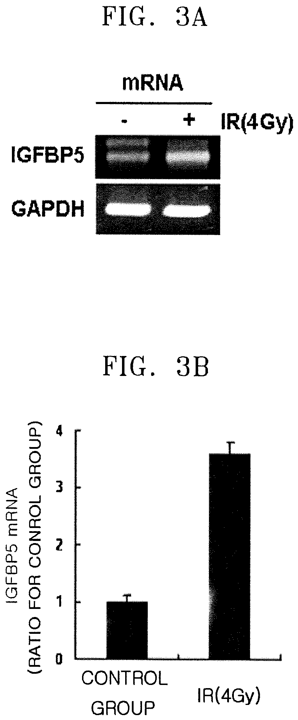

The results of the mRNA measurements and protein level measurements are shown in FIGS. 3A to 3C.

FIG. 3A is an image showing a result of quantified amounts of mRNA of IGFBP5 genes by GAPDH, wherein the mRNA is extracted from HUVECs after irradiation.

FIG. 3B is a graph showing a result of quantified amounts of mRNA of IGFBP5 genes by GAPDH, wherein the mRNA is extracted from HUVECs after irradiation.

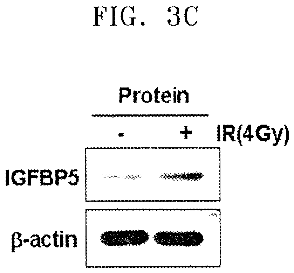

FIG. 3C is an image showing amounts of IGFBP5 protein determined by Western blotting, wherein the IGFBP5 protein is extracted from HUVECs after irradiation.

### <Example 4> Analysis of effects of irradiation to cells in which genes are overexpressed

### 1. IGFBP5 overexpression

*IGFBP5* genes were amplified by PCR and cloned in a pLenti6/v5 TOPO vector (Invitrogen, U.S.A), thereby manufacturing a Lentiviral expression vector. 293FT cells were subjected to transfection with the pLenti6/V5_IGFBP5 vector and a Packaging Mix including pLP1, pLP2, and pLP/VSVG using lipofectamine 2000 (Invitrogen). After 72 hours of the transfection, the supernatant of the 293FT cell culture medium was taken to obtain a virus therein. The obtained virus infected HUVECs, and then the infected HUVECs were irradiated for experimentation.

### 2. Cell viability analysis (MTT assay)

IGFBP5 within the cells was overexpressed due to the infection of the IGFBP5, and then, cell growth or cell viability of the cells was measured using an MTT assay in a case of the cells being irradiated with 4 Gy or the cells not being irradiated. The cells were divided into two groups, i.e., a group being irradiated in a 96-well on which the cells were dispensed, and another group not being irradiated. Then, an MTT assay was performed with respect to the two groups after 0, 48, and 96 hours of the irradiation. 5 mg/ml of MTT was added to each well, and the plate was incubated at a temperature of 37°C for 2 hours. After removing the culture medium, 300 µℓ of DMSO was added thereto. Thereafter, values at a wavelength of 595 nm were observed using a microarray plate.

In addition, proteins were extracted from a treatment group, in which the IGFBP5-overexpressed HUVECs was either irradiated or not irradiated, and then the level of IGFBP5 expression were measured using Western blotting.

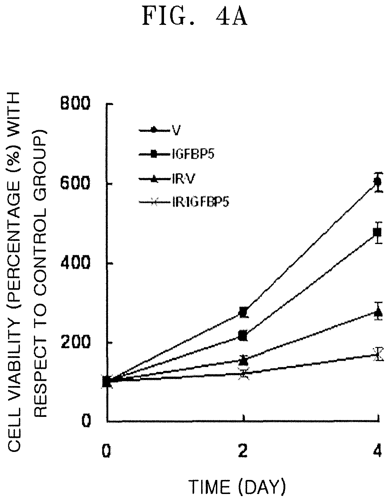

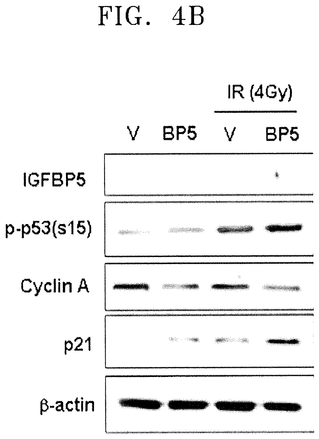

The results are shown in FIGS. 4A and 4B.

FIG. 4A is a graph showing a result of viability of cells that overexpress IGFBP5 by infection of IGFBP5 or do not overexpress IGFBP5 measured according to MTT analysis, wherein the cells are either irradiated or not irradiated with 4 Gy of radiation.

FIG. 4B is an image showing content of various proteins including IGFBP5 determined by Western blotting after extracting proteins from cells that overexpress IGFBP5 by infection of IGFBP5 or do not overexpress IGFBP5, wherein the cells are either irradiated or not irradiated with 4 Gy of radiation.

According to the results, it was confirmed that the overexpression of the IGFBP5 caused inhibition of the cell growth. In addition, it was observed that, also after the irradiation, the cell viability of the cells in which the IGFBP5 was overexpressed was significantly decreased. Therefore, it was confirmed that the overexpression of the IGFBP5 may cause cell damage, and may show synergy effects in irradiation-induced DNA damage responses.

### <Example 5> Analysis of effects of irradiation to cells in which genes are knockdown

### 1. IGFBP5 miRNA expression

IGFBP5 miRNAs were designed using Lentiviral expression systems (Invitrogen, USA). Then, HUVECs cells were dispensed thereon to be infected with Lentivirus including the IGFBP5 miRNAs. The infected HUVECs cells were used for experimentation after it was confirmed that the contents of the IGFBP5 mRNAs were decreased to less than 50% after 72 hours of the virus infection.

### 2. Cell viability analysis (MTT assay)

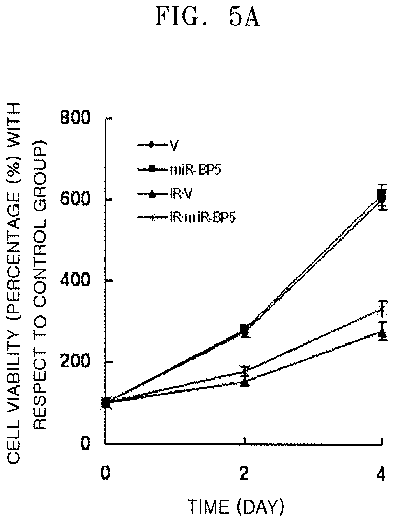

Knockdown of endogenous IGFBP5 occurred due to miRNA infection in IGFBP5, and then cell growth or viability of cells was measured using an MTT assay in a case of the cells being irradiated with 4 Gy or the cells not being irradiated. Cells in which the IGFBP5 knockdown occurred were dispensed on a 96-well plate, and then, divided into two groups each of which was irradiated and was not irradiated. MTT analysis was conducted at 0, 48, and 96 hours after the irradiation. 5 mg/ml of MTT was added to each well, and the plate was incubated at a temperature of 37°C for 2 hours. After removing the culture medium, 300 *µ*ℓ of DMSO was added thereto. Thereafter, values at a wavelength of 595 nm were obtained using a microarray. The results of cell viability obtained therefrom are shown in FIG. 5A.

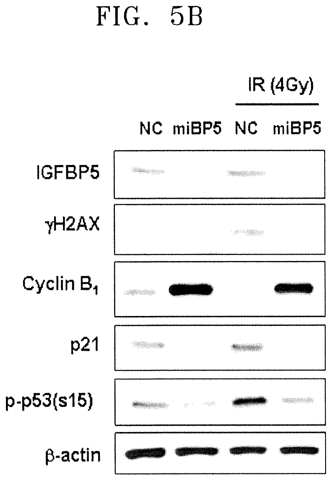

In addition, proteins were extracted from cells in an experimental group in which the cells are irradiated or not irradiated in HUVECs with the IGFBP5 knockdown. Then, content of various proteins including IGFBP5 was measured using Western blotting. The results are shown in FIG. 5B.

FIG. 5A is a graph illustrating a result of viability of cells measured according to MTT analysis, wherein the cells are either irradiated or not irradiated with 4 Gy of radiation after knockdown of the IGFBP5 due to IGFBP5 miRNA infection.

FIG. 5B is an image showing content of various proteins including IGFBP5 determined by Western blotting after extracting proteins from cells, wherein the cells are either irradiated or not irradiated with 4 Gy of radiation after knockdown of the IGFBP5 due to IGFBP5 miRNA infection.

According to the results above, it was confirmed that, as shown in FIG. 5, the reduction in cell viability due to radiation was inhibited only by the IGFBP5 knockdown. Therefore, it was confirmed that inhibition of the expression of IGFBP5 can recover inhibition of the cell growth of vascular endothelial cell due to radiation.

### 3. Capillary formation assay

Knockdown of IGFBP-5 within the cell occurred due to IGFBP5 miRNA infection, and then, angiogenic capability of cells that were irradiated or not irradiated in 4 Gy was measured.

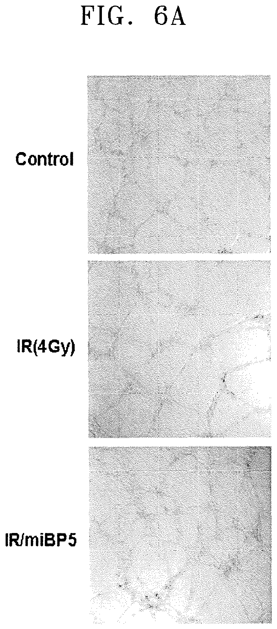

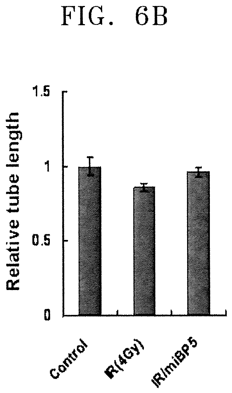

500 *µ*ℓ of Matrigel (BD Bioseieces, USA) was added to each well of a 24-well plate, and then, was hardened for 30 minutes. 1.5x10⁵ HUVECs were disposed on top of each gel and grown for 20 hours. The length of the cells formed in a tube shape was measured and the number of tube branches was counted to analyze differences in angiogenesis. The results are shown in FIGS. 6A and 6B.

FIG. 6A shows images illustrating an angiogenic status in the case of irradiation with 4 Gy of radiation to the cells in which IGFBP5 knockdown occurred due to IGFBP5 miRNA infection.

FIG. 6B is a graph showing a result of measuring the length of a blood vessel formed by irradiating the cells with 4 Gy of radiation in which IGFBP5 knockdown occurred due to miRNA infection in comparison with those of a control group.

According to the results above, degradation of angiogenesis by irradiation was found to be recovered again in the cells as much as angiogenesis in the control group, wherein the cells had occurred IGFBP5 knockdown.
## Claims

1. A method of diagnosing radiation exposure, the method comprising:
measuring expression levels of an insulin-like growth factor-binding protein-5 (IGFBP-5) gene at an mRNA or the protein in a patient's sample that is obtained from vascular endothelial cells;
comparing the measured expression levels of the *IGFBP-5* gene at an mRNA or the protein to those of a normal control group; and
determining the patient to be exposed to radiation in a case of higher expression levels in the patient's sample than to those in the normal control group.

2. A method of screening an agent for enhancing radiation sensitivity, the method comprising:
treating cells of a vascular endothelial cell line with individual samples of the agent;
measuring expression levels of an insulin-like growth factor-binding protein-5 (IGFBP-5) at an mRNA or the protein in the vascular endothelial cells; and
selecting a sample of the agent having increased expression levels of the *IGFBP-*5 gene at an mRNA or the protein compared to those of a control group.

3. A method of screening an agent for radiation protection, the method comprising:
treating cells of a vascular endothelial cell line with individual samples of the agent;
measuring expression levels of an insulin-like growth factor-binding protein-5 (IGFBP-5) gene at an mRNA or the protein in the vascular endothelial cells; and
selecting a sample of the agent having decreased expression levels of the *IGFBP-5* gene at an mRNA or the protein compared to those of a control group.

4. The method of any one of claims 1-3, wherein the mRNA expression levels is measured using a primer or a probe binding specifically to the *IGFBP-5* gene; or,
wherein the mRNA expression levels is measured using at least one method selected from RT-PCR, competitive RT-PCR, real-time RT-PCR, RNase protection assay (RPA), and Northern blotting, or a method using a DNA microarray chip; or,
wherein measuring of the protein expression levels uses an antibody binding specifically to IGFBP-5.

5. The method of any one of claims 1-3, wherein the protein expression levels is measured using at least one method selected from Western blotting, ELISA, radioimmunoassay, radioimmunodiffusion, Ouchterlony immunodiffusion, rocket immunoelectrophoresis, histoimmunostaining, immunoprecipitation assay, complement fixation assay, and FACS, or a method using a protein chip.

## Patentansprüche

1. Verfahren zum Diagnostizieren von Strahlenbelastung, wobei das Verfahren Folgendes umfasst:
Messen von Expressionswerten eines Insulin-like-Wachstumsfaktor-bindenden-Protein-5(IGFBP-5)-Gens an einer mRNA oder des Proteins in einer Probe eines Patienten, die von vaskulären Endothelzellen gewonnen worden ist;
Vergleichen der gemessenen Expressionswerte des *IGFBP-5-*Gens an einer mRNA oder des Proteins mit denen einer normalen Kontrollgruppe; und
Bestimmen, im Falle von höheren Expressionswerten in der Probe des Patienten als bei der normalen Kontrollgruppe, dass der Patient Strahlung ausgesetzt ist.

2. Verfahren zum Screenen eines Mittels zum Verbessern von Strahlensensibilität, wobei das Verfahren Folgendes umfasst:
Behandeln von Zellen einer vaskulären Endothelzelllinie mit individuellen Proben des Mittels;
Messen von Expressionswerten eines Insulin-like-Wachstumsfaktor-bindenden-Proteins-5 (IGFBP-5) an einer mRNA oder des Proteins in den vaskulären Endothelzellen; und
Auswählen einer Probe des Mittels mit erhöhten Expressionswerten des *IGFBP-5-*Gens an einer mRNA oder des Proteins im Vergleich zu denen einer Kontrollgruppe.

3. Verfahren zum Screenen eines Mittels für Strahlenschutz, wobei das Verfahren Folgendes umfasst:
Behandeln von Zellen einer vaskulären Endothelzelllinie mit individuellen Proben des Mittels;
Messen von Expressionswerten eines Insulin-like-Wachstumsfaktor-bindenden-Protein-5(IGFBP-5)-Gens an einer mRNA oder des Proteins in den vaskulären Endothelzellen; und
Auswählen einer Probe des Mittels mit verringerten Expressionswerten des *IGFBP-5-*Gens an einer mRNA oder des Proteins im Vergleich zu denen einer Kontrollgruppe.

4. Verfahren nach einem der Ansprüche 1-3, wobei die mRNA-Expressionswerte unter Verwendung eines Primers oder einer Probe, der/die spezifisch an das *IGFBP-5-*Gen bindet, gemessen werden; oder
wobei die mRNA-Expressionswerte unter Verwendung wenigstens eines Verfahrens, ausgewählt aus RT-PCR, kompetitiver RT-PCR, Echtzeit-RT-PCR, RNase-Schutzassay (*RNase protection* assay- RPA) und Northern Blotting, oder eines Verfahrens unter Verwendung eines DNA-Microarray-Chips gemessen werden; oder
wobei das Messen der Proteinexpressionswerte einen spezifisch an *IGFBP-5* bindenden Antikörper verwendet.

5. Verfahren nach einem der Ansprüche 1-3, wobei die Proteinexpressionswerte unter Verwendung wenigstens eines Verfahrens, ausgewählt aus Western Blotting, ELISA, Radioimmunassay, Radioimmundiffusion, Ouchterlony-Immundiffusion, Rocket-Immunelektrophorese, Histoimmunostaining, Immunpräzipitationsassay, Komplementbindungsassay und FACS, oder eines Verfahrens unter Verwendung eines Proteinchips gemessen werden.

## Revendications

1. Procédé de diagnostic de l'exposition aux rayonnements, le procédé comprenant les étapes consistant à :
mesurer les niveaux d'expression d'un gène de protéine 5 se liant au facteur de croissance insulinomimétique (IGFBP-5) au niveau d'un ARNm ou de la protéine dans un échantillon de patient obtenu à partir de cellules endothéliales vasculaires ;
comparer les niveaux d'expression mesurés du gène IGFBP-5 au niveau d'un ARNm ou de la protéine avec ceux d'un groupe de contrôle normal ; et
déterminer le patient à exposer aux rayonnements si les niveaux d'expression sont plus élevés dans l'échantillon du patient que dans le groupe de contrôle normal.

2. Procédé de criblage d'un agent favorisant la sensibilité aux rayonnements, le procédé comprenant les étapes consistant à :
traiter les cellules d'une lignée cellulaire endothéliale vasculaire avec des échantillons individuels de l'agent ;
mesurer les niveaux d'expression d'une protéine 5 se liant au facteur de croissance insulinomimétique (IGFBP-5) au niveau d'un ARNm ou de la protéine dans les cellules endothéliales vasculaires ; et
sélectionner un échantillon de l'agent ayant des niveaux d'expression plus élevés du gène IGFBP-5 au niveau d'un ARNm ou de la protéine que dans un groupe de contrôle.

3. Procédé de criblage d'un agent de protection contre les rayonnements, le procédé comprenant les étapes consistant à :
traiter les cellules d'une lignée cellulaire endothéliale vasculaire avec des échantillons individuels de l'agent ;
mesurer les niveaux d'expression d'une protéine 5 se liant au facteur de croissance insulinomimétique (IGFBP-5) au niveau d'un ARNm ou de la protéine dans les cellules endothéliales vasculaires ; et
sélectionner un échantillon de l'agent ayant des niveaux d'expression moins élevés du gène IGFBP-5 au niveau d'un ARNm ou de la protéine que dans un groupe de contrôle.

4. Procédé selon l'une quelconque des revendications 1 à 3, dans lequel les niveaux d'expression de l'ARNm sont mesurés à l'aide d'une amorce ou d'une sonde se liant spécifiquement au gène IGFBP-5 ; ou
dans lequel les niveaux d'expression de l'ARNm sont mesurés en utilisant au moins un procédé choisi parmi la RT-PCR, la RT-PCR compétitive, la RT-PCR en temps réel, l'essai de protection à la RNase (RPA) et le buvardage de northern, ou un procédé utilisant un micro-réseau d'ADN ;
dans lequel l'étape consistant à mesurer les niveaux d'expression de la protéine utilise un anticorps se liant spécifiquement à IGFBP-5.

5. Procédé selon l'une quelconque des revendications 1 à 3, dans lequel les niveaux d'expression de la protéine sont mesurés en utilisant au moins un procédé choisi parmi le buvardage de western, la méthode ELISA, le radioimmunodosage, la radioimmunodiffusion, l'immunodiffusion d'Ouchterlony, l'immunoélectrophorèse en roquettes,
l'immunohistocoloration, l'essai par immunoprécipitation, l'essai de fixation du complément et la FACS, ou un procédé utilisant une puce à protéines.
